# EUROPEAN PATENT APPLICATION

(11) **EP 4 249 020 A2**
(43) Date of publication of application: **27.09.2023**
(21) Application number: 23190277.6
(22) Date of filing: 28.09.2020
(51) Int. Cl.: A61M 11/06

(54) **NASAL CANNULA WITH INTEGRATED NEBULIZER**

(30) Priority: 30.09.2019 US 201962908512 P
(62) Divisional of application: 20799863.4
(71) Applicant: SunMed Group Holdings, LLC, Grand Rapids, MI 49544 (US)
(72) Inventor: VARGA, Christopher M., Mettawa, 60045 (US)
(74) Representative: Epping - Hermann - Fischer

(57) **Abstract**

Nasal cannulas are described herein. The nasal cannula includes a cannula housing, a reservoir, a nebulizer channel, and a gas channel. The cannula housing includes at least one nasal extension extending from the cannula housing, wherein the at least one nasal extension is configured to be disposed within a patient's nares. The reservoir includes a reservoir volume configured to dispense a medicament. The nebulizer channel is in fluid communication with the reservoir volume and the at least one nasal extension. The gas channel is configured to direct a gas flow toward the at least one nasal extension through the nebulizer channel, drawing a portion of the medicament from the reservoir through the nebulizer channel with the gas flow toward the at least one nasal extension.

## Description

### FIELD OF THE INVENTION

The present disclosure generally relates to nasal cannula devices, and, in particular, to nasal cannula devices with an integrated nebulizer.

### BACKGROUND

The delivery of a gas to patients, such as the delivery of air or oxygen in supplemental gas therapy, is a well-known treatment for a number of illnesses and conditions. A nasal cannula can be used to direct supplemental gas flow to a patient's nares. During respiratory therapy or support with a nasal cannula, it is often desirable to also treat a patient with therapeutic drugs or medicaments.

In some applications, during supplemental gas therapy administered via a nasal cannula, administration of therapeutic drugs can diminish the effectiveness of the supplemental gas therapy and/or the administered drugs.

### SUMMARY

The disclosed subject matter relates to nasal cannulas. In certain embodiments, a nasal cannula is disclosed that comprises a cannula housing defining a housing volume, the cannula housing comprising at least one nasal extension extending from the cannula housing, wherein the at least one nasal extension is configured to be disposed within a patient's nares; a reservoir disposed at least partially within the housing volume, wherein the reservoir comprises a reservoir volume configured to dispense a medicament; a nebulizer channel in fluid communication with the reservoir volume and the at least one nasal extension; and a gas channel in fluid communication with the nebulizer channel, wherein the gas channel is configured to direct a gas flow toward the at least one nasal extension through the nebulizer channel, drawing a portion of the medicament from the reservoir through the nebulizer channel with the gas flow toward the at least one nasal extension.

In certain embodiments, a nasal cannula is disclosed that comprises a cannula housing defining a housing volume, the cannula housing comprising at least one nasal extension extending from the cannula housing, wherein the at least one nasal extension is configured to be disposed within a patient's nares; a reservoir disposed at least partially within the housing volume, wherein the reservoir comprises a reservoir volume configured to dispense a medicament; a nebulizer channel in fluid communication with the reservoir volume and the at least one nasal extension; a gas channel in fluid communication with the nebulizer channel, wherein the gas channel is configured to direct a gas flow toward the at least one nasal extension through the nebulizer channel, drawing a portion of the medicament from the reservoir through the nebulizer channel with the gas flow and forming a nebulizing gas flow directed toward the at least one nasal extension; and a therapeutic gas channel in fluid communication with the at least one nasal extension, wherein the therapeutic gas channel is configured to direct a therapeutic gas flow toward the at least one nasal extension.

In certain embodiments, a method is disclosed that comprises introducing at least one nasal extension of a cannula housing into a patient's nares; directing a gas flow through a nebulizer channel toward the at least one nasal extension; drawing a portion of a medicament from a reservoir through the nebulizer channel with the gas flow, wherein the reservoir is disposed within the cannula housing; and directing the gas flow and the portion of the medicament toward the patient's nares via the at least one nasal extension.

In certain embodiments, a method is disclosed that comprises introducing at least one nasal extension of a cannula housing into a patient's nares; directing a gas flow through a nebulizer channel toward the at least one nasal extension; drawing a portion of a medicament from a reservoir through the nebulizer channel with the gas flow, wherein the reservoir is disposed within the cannula housing; forming a nebulizing gas flow by mixing the portion of the medicament with the gas flow; directing the nebulizing gas flow and the portion of the medicament toward the patient's nares via the at least one nasal extension; and directing a therapeutic gas flow through a therapeutic gas channel toward the patient's nares via the at least one nasal extension.

It is understood that various configurations of the subject technology will become readily apparent to those skilled in the art from the disclosure, wherein various configurations of the subject technology are shown and described by way of illustration. As will be realized, the subject technology is capable of other and different configurations and its several details are capable of modification in various other respects, all without departing from the scope of the subject technology. Accordingly, the summary, drawings and detailed description are to be regarded as illustrative in nature and not as restrictive.

### BRIEF DESCRIPTION OF THE DRAWINGS

The accompanying drawings, which are included to provide further understanding and are incorporated in and constitute a part of this specification, illustrate disclosed embodiments and together with the description serve to explain the principles of the disclosed embodiments. In the drawings:
FIG. 1A is a perspective view of a nebulizer system, in accordance with various aspects of the present disclosure.
FIG. 1B is a perspective view of a nebulizer system, in accordance with various aspects of the present disclosure.
FIG. 2 is a perspective view of the nasal cannula of FIG. 1A, in accordance with various aspects of the present disclosure.
FIG. 3 is a partial top view of the nasal cannula of FIG. 1A, in accordance with various aspects of the present disclosure.
FIGS. 4A-4C are top views of the flow restrictor of FIG. 3, in accordance with various aspects of the present disclosure.
FIG. 5 is a partial cross-sectional view of the nasal cannula of FIG. 1A, in accordance with various aspects of the present disclosure.
FIG. 6 is a partial cross-sectional view of a nebulizer system, in accordance with various aspects of the present disclosure.
FIG. 7 is a partial cross-sectional view of a nebulizer system, in accordance with various aspects of the present disclosure.
FIGS. 8A and 8B are simplified perspective views of a nebulizer system, in accordance with various aspects of the present disclosure.
FIG. 9 is a perspective view of a nebulizer system, in accordance with various aspects of the present disclosure.
FIG. 10 is a partial cross-sectional view of a nebulizer system, in accordance with various aspects of the present disclosure.
FIG. 11 is a perspective view of a nebulizer system, in accordance with various aspects of the present disclosure.
FIG. 12 is a perspective view of a nebulizer system, in accordance with various aspects of the present disclosure.

### DETAILED DESCRIPTION

The disclosed nasal cannula incorporates features to permit the administration of a medicament without diminishing the effectiveness of the supplemental gas therapy and/or the administered drugs. The nasal cannula can dispense a medicament within a flow path defined within the cannula housing to generate a medicament aerosol in the immediate vicinity of the patient's airway.

The detailed description set forth below is intended as a description of various configurations of the subject technology and is not intended to represent the only configurations in which the subject technology may be practiced. The detailed description includes specific details for the purpose of providing a thorough understanding of the subject technology. However, it will be apparent to those skilled in the art that the subject technology may be practiced without these specific details. In some instances, well-known structures and components are shown in block diagram form in order to avoid obscuring the concepts of the subject technology. Like components are labeled with identical element numbers for ease of understanding. Reference numbers may have letter suffixes appended to indicate separate instances of a common element while being referred to generically by the same number without a suffix letter.

While the following description is directed to the administration of supplemental gas and/or a medicament to a patient using the disclosed nasal cannula, it is to be understood that this description is only an example of usage and does not limit the scope of the claims. Various aspects of the disclosed nasal cannula may be used in any application where it is desirable to administer a supplemental gas and/or a medicament.

The disclosed nasal cannula overcomes several challenges discovered with respect to certain medication delivery devices. One challenge with certain conventional medication delivery devices, such as nebulizers, metered-dose inhalers (MDIs), and dry-powder inhalers is that such devices are primarily configured for mouth use and are not convenient to use for nose-breathing patients (such as infants, children and many adults), and further are not convenient during nasal gas therapy wherein the airways are flushed and mildly pressurized with a gas (e.g. oxygen) at a therapeutic flow rate. As a result, nasal cannulas are often removed during medication delivery, which may disturb the respiratory therapy and complicate caregiver workflow. Another challenge with certain conventional medication delivery devices, such as nebulizers (including vibrating mesh nebulizers) and atomizers, is that such devices will generate a soft mist of medicament droplets or particles upstream of a nasal cannula that can be lost to the walls or cannula surfaces as the medicament moves toward the patient's nasal prongs. Further, another challenge with certain medication delivery devices is that such devices are disposed near a humidifier in the breathing circuit, causing medicament particle growth and/or modification of the medicament particles.

Therefore, in accordance with the present disclosure, it is advantageous to provide a nasal cannula as described herein that allow for the administration of medicament during nasal gas (e.g. oxygen) therapy. In some embodiments, the nasal cannula can allow for the administration of medicament during high-flow nasal gas therapy. Further, it is advantageous to provide a nasal cannula that generates a medicament aerosol within the nasal cannula and away from a humidity source to prevent the loss of medicament to cannula surfaces, the growth of medicament particles, and/or the modification of medicament particles.

Examples of nasal cannulas that allow for the administration of medicament during nasal gas therapy are now described.

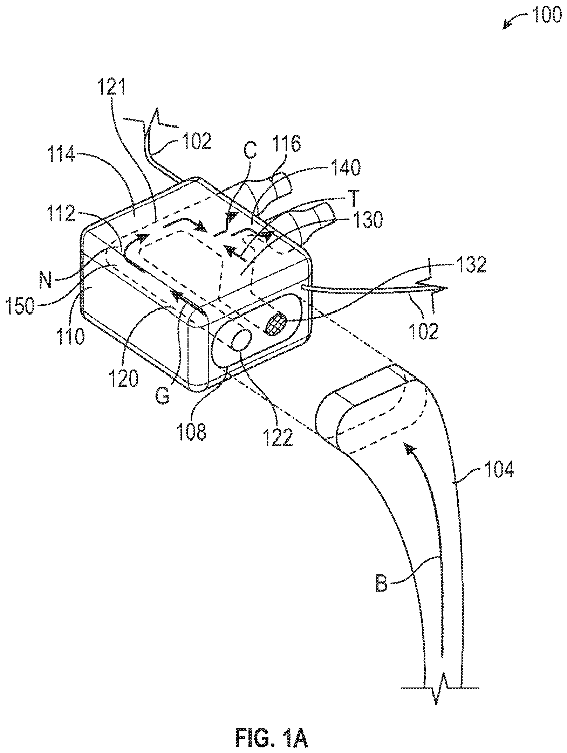

FIG. 1A is a perspective view of a nebulizer system 100 with simplified internal features illustrated, in accordance with various aspects of the present disclosure. In the depicted example, the nebulizer system 100 includes a nasal cannula 110 that delivers or administers supplemental gas flow with a medicament aerosol to a patient's nares. Medicaments can include, but are not limited to, surfactants, drugs (e.g. antibiotics, anti-viral, etc.), polymers, naturopathic remedies and/or homeopathic remedies. The nasal cannula 110 can include a housing volume 114, a gas channel port 122, and a therapeutic gas channel port 132. A gas channel 120 extends within the nasal cannula 110 from the gas channel port 122, and a therapeutic gas channel 130 extends within the nasal cannula 110 from the therapeutic gas channel port 132.

As illustrated, the nasal cannula 110 receives bulk flow B of gas (e.g. air, oxygen, and/or another gas or combination thereof) from a supply tube 104. Bulk flow B can flow into the gas channel 120 via the gas channel port 122 and/or the therapeutic gas channel 130 via the therapeutic gas channel port 132. As described herein, the bulk flow B can be directed only to the gas channel 120 or to a combination of the gas channel 120 and the therapeutic gas channel 130. As can be appreciated, the gas channel 120 and/or the therapeutic gas channel 130 can be defined or formed within the housing volume 114 of the cannula housing 112. Optionally, the cannula housing 112 can include a tubing engagement portion 108 such that the supply tube 104 can couple to the cannula housing 112 using the tubing engagement portion 108.

In the depicted example, gas flow G received by the gas channel 120 can be utilized by a nebulizer engine 150 to generate medicament aerosol or nebulizer flow N within the housing volume 114 to be directed to or otherwise administered to the patient. As described herein, the nebulizer engine 150 can be a pneumatic aerosol generator that utilizes accelerated flow to draw medicament out of a reservoir and pulverizes the medicament into an aerosol. Optionally, the nasal cannula 110 can include one or more nebulizer engines 150 with multiple respective gas channels 120 to generate aerosol within the nasal cannula 110. Advantageously, by utilizing a nebulizer engine 150 within the housing volume 114, the medicament aerosol or nebulizer flow N is generated in the immediate vicinity of the point of gas flow entry into the patient's airway (nares).

Optionally, therapeutic gas flow T received by the therapeutic gas channel 130 can be directed toward the patient, bypassing the nebulizer engine 150. In some embodiments, therapeutic gas flow T can remix or recombine with the nebulized gas flow N in a combining volume 140, to form a combined flow C prior to being delivered to the patient. As can be appreciated, in some embodiments, the entire bulk flow B may pass through the gas channel 120 and the nebulizer engine 150, bypassing the therapeutic gas channel 130. Optionally, the therapeutic gas channel 130 may be omitted from the nebulizer system 100.

During operation, gas flow G from the gas channel 120 and/or therapeutic gas flow T from the therapeutic gas channel 130 can be directed into the patient's nares through nasal extensions 116 extending from the cannula housing 112. As can be appreciated, the nasal extensions 116 can be generally shaped or formed to conform to the anatomy of the patient. As illustrated, the nasal extensions 116 are generally in fluid communication with the housing volume 114. In some embodiments, the nasal extensions 116 are in fluid communication with the gas channel 120 and/or the therapeutic gas channel 130. Optionally, the nasal extensions are in fluid communication with the combining volume 140.

As can be appreciated, the nasal cannula 110 can be disposed adjacent to a patient's nares, allowing the nasal extensions 116 to extend into the patient's nares. Optionally, the nasal cannula 110 can be secured to the patient with a strap 102 that can be placed around the patient's head, securing the nasal cannula 110 in a desired position.

During operation, bulk flow B during therapy with nasal cannula 110 can range from 1-3 L/min up to 60 L/min and in some cases even higher values such as greater than 100 L/min. In some embodiments, bulk flow B can range between approximately 1 L/min to 10 L/min, approximately 1 L/min to 7 L/min, approximately 1 L/min to 5 L/min, and approximately 1 L/min to 3 L/min. In some embodiments, bulk flow B can range between approximately 1 L/min to 150 L/min, approximately 1 L/min to 120 L/min, approximately 1 L/min to 100 L/min, approximately 1 L/min to 80 L/min, and approximately 1 L/min to 60 L/min.

As can be appreciated, in some embodiments, the gas flow G through the nebulizer engine 150 may be limited to a smaller range to provide for increased efficiency and/or consistent droplet size production. For example, in some embodiments, the gas flow G may be limited to a range between approximately 2 L/min to approximately 10 L/min to provide for increased efficiency and/or consistent droplet size production. In some embodiments, the gas flow G can range between approximately 1 L/min to 20 L/min, approximately 1 L/min to 15 L/min, approximately 1 L/min to 12 L/min, approximately 1 L/min to 10 L/min, approximately 2 L/min to 20 L/min, approximately 2 L/min to 15 L/min, approximately 2 L/min to 12 L/min, approximately 2 L/min to 8 L/min, and approximately 2 L/min to 5 L/min.

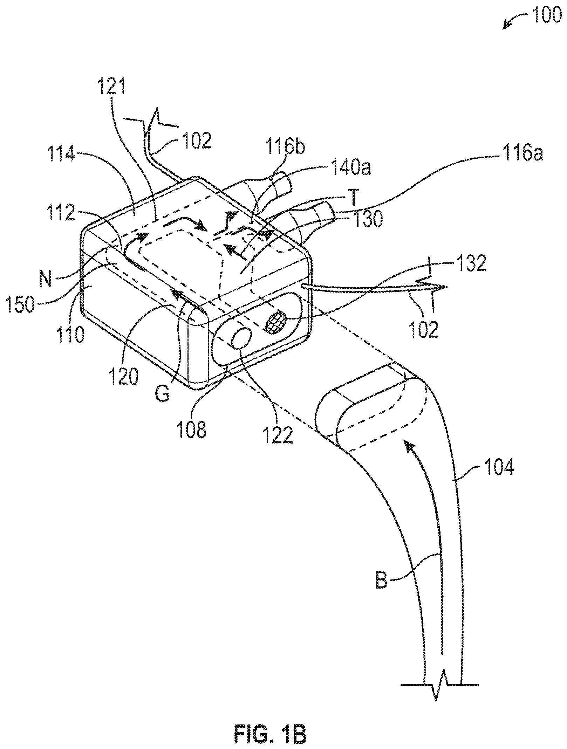

FIG. 1B is a perspective view of a nebulizer system 100, in accordance with various aspects of the present disclosure. In some embodiments, therapeutic gas flow T in the therapeutic gas channel 130 can be directed into a single nare of a patient through a single nasal extension 116a, bypassing the nebulizer engine 150. Similarly, nebulized gas flow N in the gas channel 120 can be directed into the other nare of the patient through a single nasal extension 116b. In some embodiments, therapeutic gas flow T can be separated from the nebulized gas flow N by a separating wall 140a.

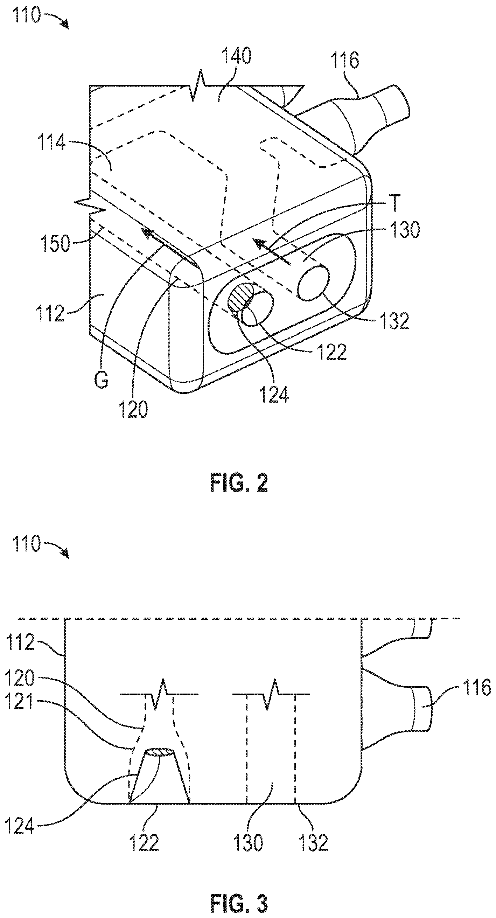

FIG. 2 is a perspective view of the nasal cannula 110 of FIG. 1A with simplified internal features illustrated, in accordance with various aspects of the present disclosure. FIG. 3 is a partial top view of the nasal cannula 110 of FIG. 1A, with simplified internal features illustrated, in accordance with various aspects of the present disclosure. With reference to FIGS. 2 and 3, in the depicted example, the nasal cannula 110 includes flow restrictions to divide or control the volume of bulk flow B that is directed toward the gas channel 120 and the therapeutic gas channel 130. A flow restriction, such as a flow restrictor 124, can divert a portion of the bulk flow B from the gas channel 120 toward the therapeutic gas channel 130. The flow restriction can provide efficient operation of the nebulization engine 150 while providing the desired amount of bulk flow B to the patient. As can be appreciated, divided or diverted portions of the flow can be rejoined in the combining volume 140 prior to administration to the patient.

In some embodiments, a fixed geometric split or relationship can be utilized to proportionally split, divide, or divert the bulk flow between the gas channel 120 and the therapeutic gas channel 130. For example, the gas channel 120 can have a cross-sectional area that is smaller than the cross-sectional area of the therapeutic gas channel 130 to direct proportionally less flow through the gas channel 120. In some applications, the nasal cannula 110 can have a 9:1 ratio between the cross-sectional area of the therapeutic gas channel 130 and the gas channel 120, such that at 20 L/min of bulk flow B, approximately 2 L/min will pass through the gas channel 120 and 18 L/min will pass through the therapeutic gas channel 130. Similarly, at a total flow of 80 L/min, the flows can be divided into 8 L/min and 72 L/min for gas channel 120 and therapeutic gas channel 130 respectively. In some embodiments, the ratio between the cross-sectional area of the therapeutic gas channel 130 and the gas channel 120 can range between 4:1, 5:1, 6:1, 7:1, 8:1, 9:1, to 10:1.

In some embodiments, a pressure relief mechanism can be used to divide or divert flow between the gas channel 120 and the therapeutic gas channel 130 such that all flow is initially directed toward the gas channel 120 until a desired flow rate is achieved and the flow is then divided or diverted. For example, optionally, a pressure relief valve can be disposed within the therapeutic gas channel 130. In some embodiments, the pressure relief valve is disposed at or near the therapeutic gas channel port 132. During operation, bulk flow B delivered to the nasal cannula 110 can pass to the gas channel 120 until a threshold pressure associated with a target gas flow G rate (e.g. 2 L/min to 10 L/min) is reached in the gas channel. When the target pressure is exceeded, the pressure relief valve opens a communicating channel into the therapeutic gas channel 130 such that at higher bulk flow B rates, the bulk flow B is split between the gas channel 120 and the therapeutic gas channel 130. Optionally, the pressure relief valve can include any suitable mechanism, such as springs, poppets, balls, or other pressure-compensating members.

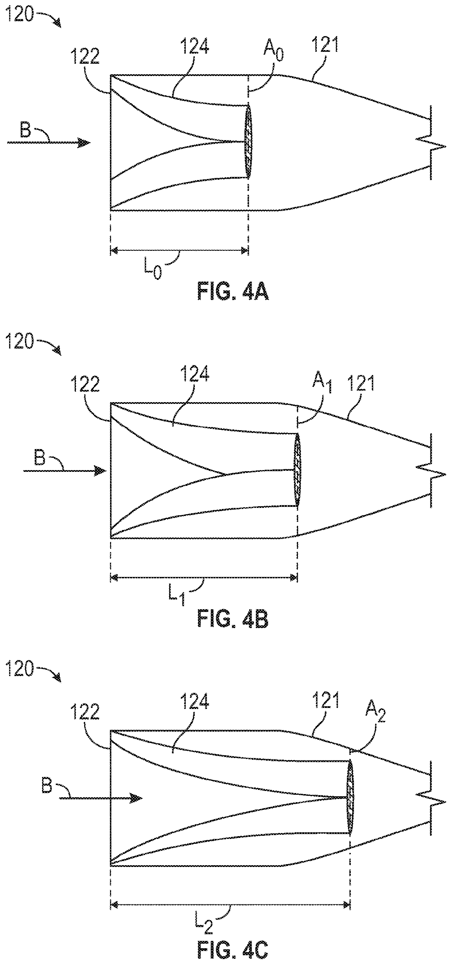

FIGS. 4A-4C are top views of the flow restrictor 124 of FIG. 3, in accordance with various aspects of the present disclosure. With reference to FIGS. 2-4C, the nasal cannula 110 can include a variable flow restrictor 124 to allow higher gas flow G rates at lower bulk flow B rates while providing an upper limit to the gas flow G rate that can flow through the gas channel 120.

In some embodiments, the flow restrictor 124 can vary the cross-sectional flow area into the gas channel 120 in response to the bulk flow B rate (or associated pressure), which can self-limit the gas flow G rate through the gas channel 120 and the nebulizer engine 150 (e.g., FIGS. 1 and 2). In the depicted example, the flow restrictor 124 is movable within a converging portion 121 of the gas channel port 122 to provide a varying cross-sectional flow area or restriction into the gas channel 120. The flow restrictor 124 can be an extendable or deformable member that extends further into the converging portion 121 in response to increased fluid pressure. The flow restrictor 124 can be formed from an elastomeric material. In some embodiments, the flow restrictor 124 can have a converging frustoconical shape that nests or otherwise extends into the converging portion 121.

For example, with reference to FIG. 4A, when no pressure is experienced by the flow restrictor 124, such as when no bulk flow B is introduced to the gas channel 120, the flow restrictor 124 can extend to a resting or initial length L₀ into the gas channel port 122. As illustrated, at the initial length L₀, the flow restrictor 124 can provide an initial cross-sectional flow area A₀, that provides minimal flow restriction of the bulk flow B into the gas channel 120. Optionally, the ratio between the cross-sectional area of the therapeutic gas channel 130 (e.g., FIGS. 1-3) and the initial cross-sectional flow area A₀ into the gas channel 120 can range between 1:1, 2:1, 3:1, 4:1, to 5:1.

With reference to FIG. 4B, as the bulk flow B rate (and the pressure) increases, the flow restrictor 124 can extend to a length L₁ into the converging portion 121 of the gas channel port 122 in response to the increased fluid pressure. As illustrated, at the length L₁, the flow restrictor 124 can provide a reduced cross-sectional flow area A₁ compared to the initial cross-sectional area A₀, increasing the flow restriction of the bulk flow B into the gas channel 120. Optionally, the ratio between the cross-sectional area of the therapeutic gas channel 130 and the cross-sectional flow area A₁ into the gas channel 120 can range between 2:1, 3:1, 4:1, 5:1, 6:1, 7: 1, to 8:1.

With reference to FIG. 4C, as the bulk flow B rate (and the pressure) further increases, the flow restrictor 124 can extend to a length L₂ into the converging portion 121 of the gas channel port 122 in response to the further increased fluid pressure. As illustrated, at the length L₂, the flow restrictor 124 can provide further reduced cross-sectional flow area A₂ compared to the cross-sectional area A₁, further increasing the flow restriction of the bulk flow B into the gas channel 120 as bulk flow B is increased. Advantageously, the use of the flow restrictor 124 allows for flow through the gas channel 120 to be limited to a maximum target flow for nebulization while allowing for a desired bulk flow B to the patient. Optionally, the ratio between the cross-sectional area of the therapeutic gas channel 130 and the cross-sectional flow area A₂ into the gas channel 120 can range between 5:1, 6:1, 7:1, 8:1, 9:1, to 10:1.

For example, at a bulk flow rate of 40 L/min, the flow restrictor 124 can provide a 4:1 ratio between the cross-sectional area of the therapeutic gas channel 130 and the cross-sectional flow area A₁ into the gas channel 120, such that at 40 L/min of bulk flow B, approximately 8 L/min will pass through the gas channel 120 and 32 L/min will pass through the therapeutic gas channel 130. As can be appreciated, the same 4:1 ratio at a higher bulk flow B rate of 80 L/min may not allow for desired operation of the nebulization system 100. Advantageously, at higher flow rates, such as 80 L/min, the flow restrictor 124 can extend to a length L₂ into the converging portion 121 of the gas channel port 122. Therefore, at a bulk flow rate of 80 L/min, the flow restrictor 124 can provide a 9:1 ratio between the cross-sectional area of the therapeutic gas channel 130 and the cross-sectional flow area A₂ into the gas channel 120, such that approximately 8 L/min will pass through the gas channel 120 and 72 L/min will pass through the therapeutic gas channel 130, allowing for increased overall bulk flow B toward the patient, while limiting gas flow G into the nebulizing engine 150.

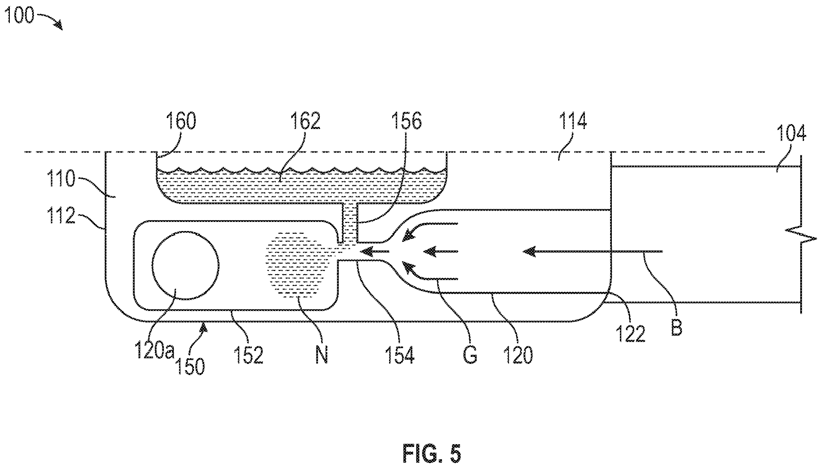

FIG. 5 is a partial cross-sectional view of the nasal cannula 110 of FIG. 1A, in accordance with various aspects of the present disclosure. In the depicted example, the nebulization engine 150 creates nebulized gas flow N from medicament 162 within the housing volume 114. In some embodiments, the medicament 162 can include liquid medicaments and/or dry powder medicaments.

As illustrated, the medicament 162 can be received, stored, and/or contained in a reservoir volume of a reservoir 160. Optionally, medicament 162 can be introduced or metered into the reservoir 160 through a medicament delivery line. In some embodiments, the reservoir 160 can include an access port to allow manual administration of medicament 162 into the reservoir 160. Further, the access port can allow for the introduction of ampules of medicament. In some embodiments, the reservoir 160 can be disposed at least partially or completely within the housing volume 114. As can be appreciated, the reservoir 160 can be separate or integrally formed with the cannula housing 112.

As described herein, the nebulization engine 150 can utilize gas flow G to administer the medicament 162 as nebulized gas flow N to the patient. During operation, the nebulization engine 150 directs gas flow G from the gas channel 120 into the nebulizing chamber 152 via a nebulizing channel 154. As illustrated, the nebulizing channel 154 can have a constricting geometry or otherwise reduced diameter relative to the gas channel 120 to increase the velocity of the gas flow G entering the nebulizing chamber 152. Optionally, the gas channel 120 can converge to the diameter of the nebulizing channel 154. As can be appreciated, as the velocity of the gas flow G is increased within the nebulizing channel 154, the fluid pressure of the gas flow G within the nebulizing channel 154 is reduced (e.g., a Venturi effect).

As a result of the reduced pressure within the nebulizing channel 154, medicament 162 from the reservoir 160 can be drawn into the nebulizing channel 154 through a reservoir channel 156. As described herein, the reservoir 160 can be disposed above, below, or laterally relative to the nebulizing channel 154. As the medicament 162 is drawn into the nebulizing channel 154 and directed into the nebulizing chamber 152 by the gas flow G, the force of the high-speed gas flow G can accelerate and shear the medicament 162, forming droplets of medicament 162 which combine with the gas flow G to form the nebulizing gas flow N. As described herein, the flow rate of the gas flow G can be controlled to permit the desired formation of medicament 162 droplets or aerosol appropriate for delivery into the patient's airways and/or lungs.

As illustrated, nebulizing gas flow N created by the nebulizing engine 150 can be directed toward the patient through a secondary gas channel 120a. In some embodiments, the secondary gas channel 120a can direct the nebulizing gas flow N directly to the nasal extensions 116 formed in the cannula housing 112. Optionally, the secondary gas channel 120a can direct the nebulizing gas flow N toward the combining volume 140 (e.g., FIGS. 1 and 2) to be combined with the therapeutic gas flow T (e.g., the remainder of the bulk flow B) prior to being directed to the patient.

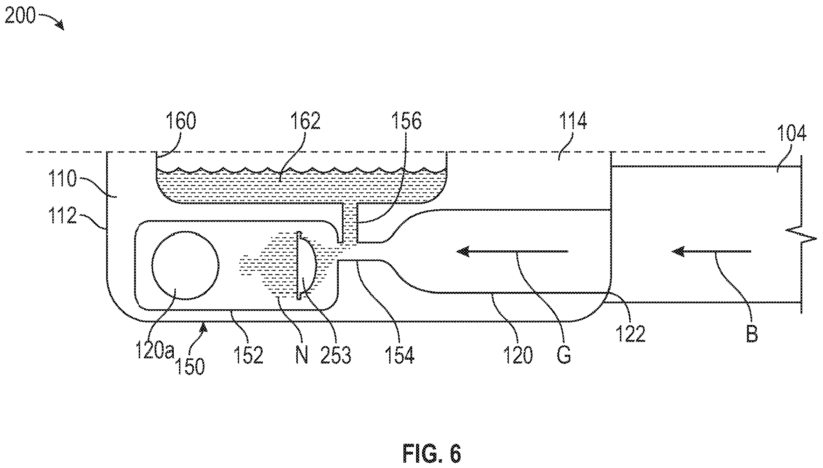

FIG. 6 is a partial cross-sectional view of a nebulizer system 200, in accordance with various aspects of the present disclosure. In the depicted example, the nebulizer system 200 includes a baffle 253 to promote disintegration of the liquid medicament 162 within the nebulizing gas flow N.

As illustrated, the baffle 253 can be disposed within the nebulizing chamber 152 such that nebulizing gas flow N impinges upon the baffle 253. The baffle 253 can be positioned between the nebulizing channel 154 and the secondary gas channel 120a. In some embodiments, at least one baffle 253 can be disposed adjacent to the exit of the nebulizing channel 154. Optionally, the baffle 253 can be spaced apart from the exit of the nebulizing channel 154. As can be appreciated, the positioning and the geometry of the baffle 253 can be adjusted relative to the nebulizing channel 154 and/or the nebulizing chamber 152 to promote droplet formation and additional pulverization within the nebulizing gas flow N.

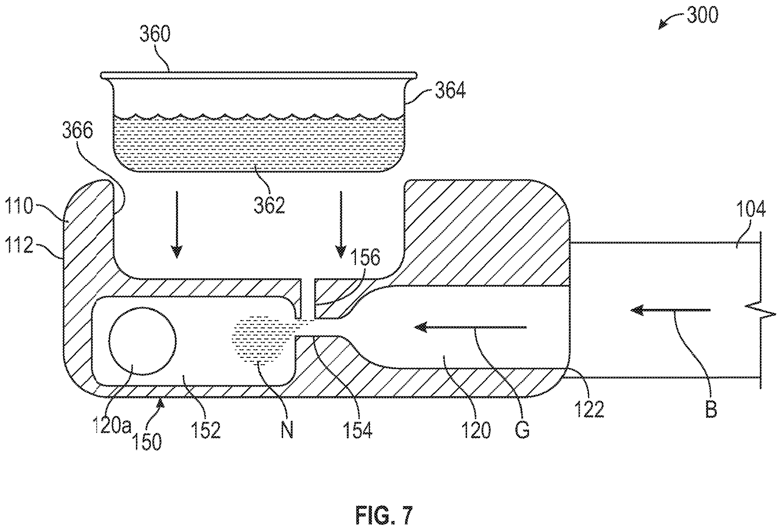

FIG. 7 is a partial cross-sectional view of a nebulizer system 300, in accordance with various aspects of the present disclosure. In the depicted example, the nebulizer system 300 allows for a removable reservoir 360 to be introduced or removed from the nasal cannula 110.

As illustrated, the removable reservoir 360 includes a pod structure or body 364 defining the reservoir volume to store the medicament 362 therein. Further, the nasal cannula 110 can include a reservoir receptacle 366 to receive and/or engage with the body 364 of the reservoir 360. Optionally, the reservoir 360 can be releasably attached or secured to the reservoir receptacle 366.

Upon docking or attaching the reservoir 360 to the nasal cannula 110, the liquid or powder medicament 362 disposed within the reservoir 360 can be exposed to the nebulizer engine 150 as described herein. In some embodiments, a clinician can open a portion of the reservoir 360 prior to docking or coupling the reservoir 360 with the nasal cannula 110. Optionally, reservoir 360 and/or the reservoir receptacle 366 may include features to automatically expose the medicament 362 within the reservoir 360 to the nebulizer engine 150 upon insertion of the reservoir 360. For example, the reservoir receptacle 366 may include a mechanism to pierce or remove a portion of the body 364 to access or expose the medicament 362 within the reservoir 360.

Advantageously, the use of removable reservoirs 360 with the nasal cannula 110 allows for rapid refilling or exchanging of medicaments 362. Further, the use of removable reservoirs 360 allows for easier configuration of the nebulizer system 300.

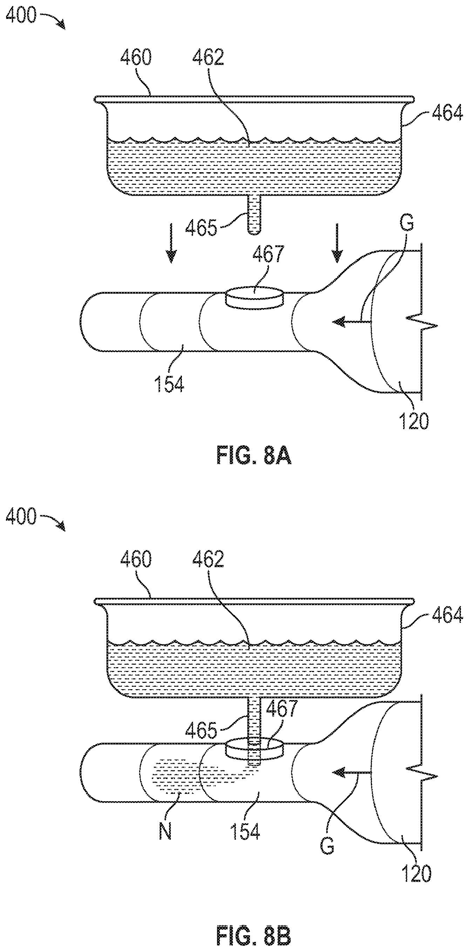

FIGS. 8A and 8B are a simplified perspective views of a nebulizer system 400, in accordance with various aspects of the present disclosure. In the depicted example, the nebulizer system 400 can include a sealing membrane 467 to allow medicament 462 stored in removable reservoirs 460 to be drawn into the nebulizer channel 154 while sealing the nebulizer channel 154 when the reservoir 460 is removed.

As illustrated in FIG. 8A, the nebulizer channel 154 can include an opening to permit the introduction of medicament 462 into the nebulizer channel 154 and the nebulizer engine generally. However, to prevent gas flow G from leaking through the opening in the nebulizer channel 154, the nebulizer channel 154 can include a sealing membrane 467 to cover the opening and allow flow to pass through the nebulizer channel 154 without leaking through the opening when a reservoir 460 is not engaged therewith. Advantageously, by utilizing the sealing membrane 467, therapeutic gas flow T to the patient can continue when reservoirs 460 are removed or exchanged.

As illustrated in FIG. 8B, the reservoir 460 can be at least partially engaged with the nebulizer channel 154 to permit medicament 462 to be drawn into the nebulizer channel 154 and into the nebulizer engine generally. In some embodiments, the reservoir 460 includes a prong 465 extending from the body 464 configured to pierce or extend through the sealing membrane 467 to allow gas flow G to access medicament 462 within the reservoir 460. As can be appreciated, a passage through the prong 465 can be opened or exposed prior to inserting the prong 465 into the nebulizer channel 154. Optionally, the passage through the prong 465 can be opened or exposed automatically by the action of docking the reservoir 460 with the nebulizer channel 154.

Optionally, the sealing membrane 467 can be a self-sealing or otherwise a multi-use membrane that allows the prong 465 or needles to be introduced or removed multiple times while retaining a seal with the nebulizer channel 154. Advantageously, the sealing membrane 467 allows for extended or prolonged treatment using a nasal cannula as reservoirs 460 can be exchanged at required intervals without interrupting the nasal therapy. In some embodiments, the sealing membrane 467 can be temporarily removed to allow the clinician to manually dispose a liquid or dry powder medicament directly into the nebulizer channel 154.

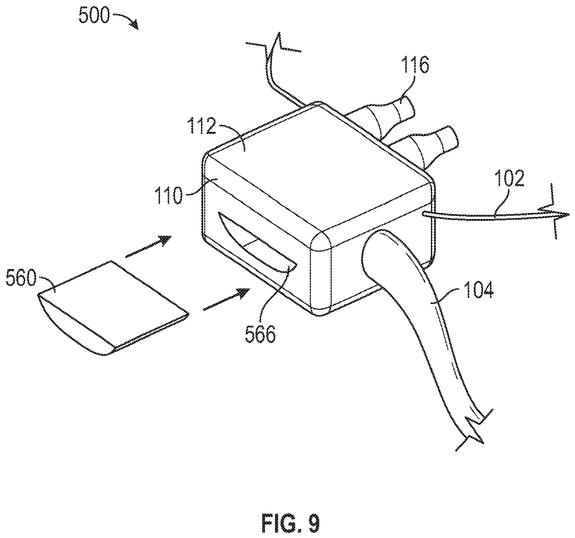

FIG. 9 is a perspective view of a nebulizer system 500, in accordance with various aspects of the present disclosure. In the depicted example, removable pods or reservoirs 560 containing medicaments can be inserted into the reservoir receptacle 566. In some embodiments, the reservoir 560 can be inserted into a side of the cannula housing 112.

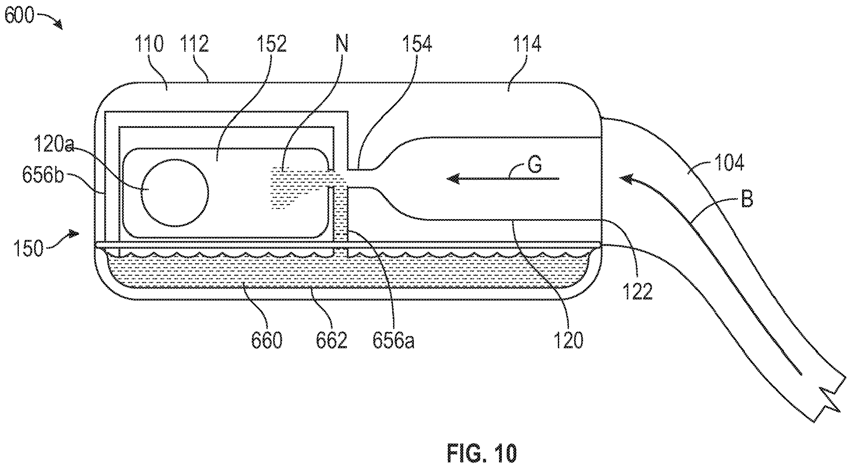

FIG. 10 is a partial cross-sectional view of a nebulizer system 600, in accordance with various aspects of the present disclosure. In the depicted example, the reservoir 660 can be disposed below the nebulizer engine 150.

As illustrated, medicament 662 can be stored or contained in a reservoir 660 below the nebulizer chamber 152 and/or the nebulizer engine 150 generally. Advantageously, by locating the reservoir 660 below the nebulizer engine 150, the cannula housing 112 may be configured to be more compact.

During operation, as a result of reduced pressure within the nebulizing channel 154, medicament 662 can be drawn into the nebulizing channel 154 though a reservoir channel 656a. As can be appreciated, the low pressure created by the gas flow G (e.g., a Venturi effect) is sufficient to draw the medicament 662 upward into the nebulizing channel 154. As previously described, as the medicament 662 is drawn into the nebulizing channel 154 and directed into the nebulizing chamber 152 by the gas flow G, the force of the high-speed gas flow G can accelerate and shear the medicament 662, forming droplets of medicament 662 which combine with the gas flow G to form the nebulizing gas flow N.

Optionally, the nebulizer system 600 can include multiple or alternative reservoir channels, such as reservoir channel 656a and 656b, that provide multiple points of fluid communication between the reservoir 660 and the nebulizing channel 154. Therefore, as medicament 662 may move or shift within the reservoir 660, the medicament 662 can be drawn into the nebulizing channel 154 by at least one of the reservoir channels 656a and 656b. As can be appreciated, the reservoir channel 656a and 656b can be spaced apart or otherwise located in various portions of the reservoir 660. Advantageously, as the patient moves into various positions and consequently moves the nasal cannula 110, multiple reservoir channels 656a and 656b can ensure that the medicament 662 maintains fluid communication with the nebulizing channel 154.

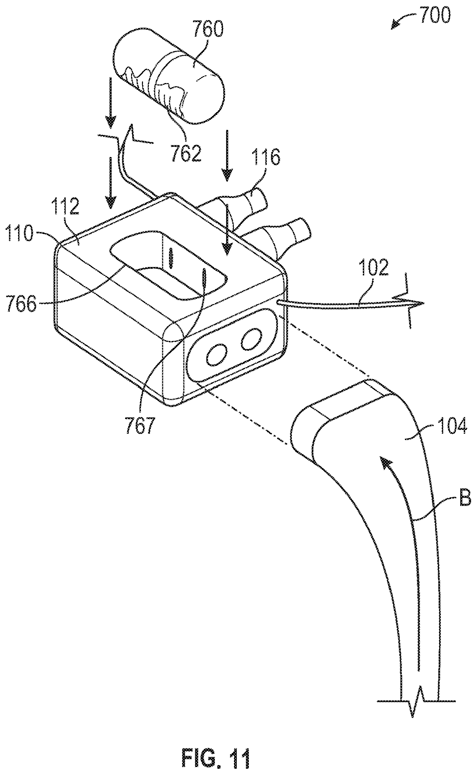

FIG. 11 is a perspective view of a nebulizer system 700, in accordance with various aspects of the present disclosure. In the depicted example, the nebulizer system 700 can nebulize or aerosolize dry powder medicaments 762.

In some embodiments, dry powder medicament 762 can be stored or contained in blisters or capsules 760. As illustrated, the capsule 760 can be inserted into a capsule receptacle 766 formed within the cannula housing 112.

Optionally, by inserting the capsule 760 into the capsule receptacle 766, one or more openings may be formed in the capsule 760 to allow medicament 762 to be drawn into the nebulization engine. An opening can be formed in the capsule 760 by a portion of the nasal cannula 110 configured to pierce, slice, or otherwise provide access to the medicament 762 of the capsule. For example, the nasal cannula 110 can include one or more projections 767 such as a needle or prong, that extends into the capsule receptacle 766. When the capsule 760 is moved into the capsule receptacle 766, the projection 767 is received within the capsule 760 and the medicament 762 can move or be drawn into the nebulization engine.

In some embodiments, the capsule 760 can be rigidly fixed or movable within the capsule receptacle 766. Optionally, the capsule 760 may shake, rattle, spin, or otherwise be agitated to release and dispense the medicament 762 therein.

As described herein, the nebulizing engine can draw the medicament 762 from the capsule 760, wherein the force of the gas flow can cause the dry or powdered medicament 762 to be disintegrated and aerosolized for delivery into the patient's airways and lungs. Advantageously, by delivering dry medicaments 762 through the nebulization system 700, wastage of medicament may be reduced.

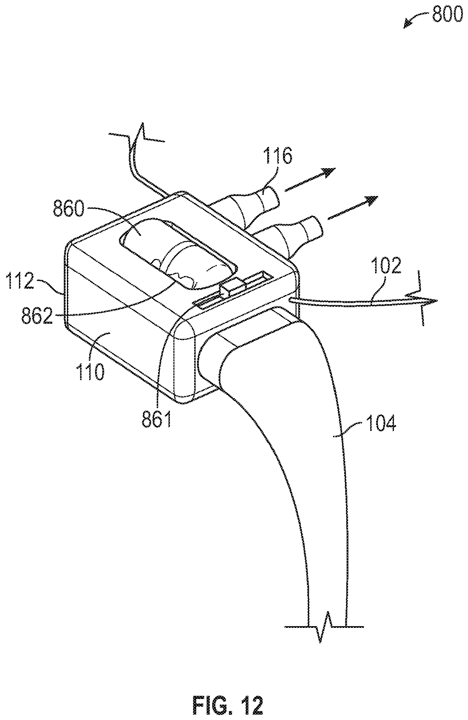

FIG. 12 is a perspective view of a nebulizer system 800, in accordance with various aspects of the present disclosure. In the depicted example, one or more openings may be formed in a capsule 860 by triggering an actuator 861.

Similar to features described with respect to nebulizer system 700, the capsule 860 can be inserted into the cannula housing 112. In some embodiments, by triggering the actuator 861, one or more openings may be formed in the capsule 860 to allow medicament 862 to be drawn in to the nebulization engine. As can be appreciated, the actuator 861 can be a button, sliding member, a spring release, or any other suitable mechanism that can form openings to allow medicament to be drawn into the nebulization engine. In some embodiments, openings in the capsule 860 can be formed by piercing, cracking, slicing, opening, and/or splitting the capsule 860.

### Illustration of Subject Technology as Clauses

Various examples of aspects of the disclosure are described as numbered clauses (1, 2, 3, etc.) for convenience. These are provided as examples, and do not limit the subject technology. Identifications of the figures and reference numbers are provided below merely as examples and for illustrative purposes, and the clauses are not limited by those identifications.

Clause 1. A nasal cannula, comprising: a cannula housing defining a housing volume, the cannula housing comprising at least one nasal extension extending from the cannula housing, wherein the at least one nasal extension is configured to be disposed within a patient's nares; a reservoir disposed at least partially within the housing volume, wherein the reservoir comprises a reservoir volume configured to dispense a medicament; a nebulizer channel in fluid communication with the reservoir volume and the at least one nasal extension; and a gas channel in fluid communication with the nebulizer channel, wherein the gas channel is configured to direct a gas flow toward the at least one nasal extension through the nebulizer channel, drawing a portion of the medicament from the reservoir through the nebulizer channel with the gas flow toward the at least one nasal extension.

Clause 2. The nasal cannula of Clause 1, wherein the nebulizer channel comprises a cross-sectional area that is smaller than a cross-sectional area of the gas channel.

Clause 3. The nasal cannula of Clause 1 or 2, wherein the reservoir is removable from the housing volume.

Clause 4. The nasal cannula of Clause 3, wherein the reservoir can be releasably attached to the cannula housing.

Clause 5. The nasal cannula of any preceding Clause, wherein the nebulizer channel comprises a sealing membrane.

Clause 6. The nasal cannula of any preceding Clause wherein the reservoir is in fluid communication with a medicament delivery line, wherein the medicament delivery line is configured to deliver medicament into the reservoir volume.

Clause 7. The nasal cannula of any preceding Clause, wherein the reservoir comprises an access port configured to allow manual administration of the medicament into the reservoir volume.

Clause 8. The nasal cannula of Clause 7, wherein the access port is configured to allow introduction of an ampule into the reservoir volume.

Clause 9. The nasal cannula of any preceding Clause, wherein the medicament comprises a liquid medicament.

Clause 10. The nasal cannula of any preceding Clause, wherein the medicament comprises a powdered medicament.

Clause 11. The nasal cannula of any preceding Clause, further comprising a therapeutic gas channel in fluid communication with the at least one nasal extension, wherein the therapeutic gas channel is configured to direct a therapeutic gas flow toward the at least one nasal extension.

Clause 12. The nasal cannula of Clause 11, wherein the gas channel comprises a flow restrictor to proportion flow between the gas channel and the therapeutic gas channel.

Clause 13. The nasal cannula of Clause 12, wherein the flow restrictor comprises an elastomeric member configured to extend at least partially into a tapered portion of the gas channel to adjustably restrict flow in the gas channel.

Clause 14. The nasal cannula of Clause 12, wherein the flow restrictor is configured to proportion flow between the therapeutic gas channel and the gas channel at a ratio of between 4:1 to 10:1.

Clause 15. The nasal cannula of Clause 11, further comprising a pressure relief valve in fluid communication with the therapeutic gas channel.

Clause 16. The nasal cannula of any preceding Clause, wherein the gas channel comprises a baffle, and wherein the gas flow impinges upon the baffle.

Clause 17. The nasal cannula of any preceding Clause, wherein the cannula housing is configured to be disposed adjacent to the patient's nares.

Clause 18. The nasal cannula of Clause 17, further comprising a strap coupled to the cannula housing, wherein the strap is configured to be disposed around a patient's head.

Clause 19. A nasal cannula, comprising: a cannula housing defining a housing volume, the cannula housing comprising at least one nasal extension extending from the cannula housing, wherein the at least one nasal extension is configured to be disposed within a patient's nares; a reservoir disposed at least partially within the housing volume, wherein the reservoir comprises a reservoir volume configured to dispense a medicament; a nebulizer channel in fluid communication with the reservoir volume and the at least one nasal extension; a gas channel in fluid communication with the nebulizer channel, wherein the gas channel is configured to draw a portion of the medicament from the reservoir through the nebulizer channel with the gas flow and form a nebulizing gas flow directed toward the at least one nasal extension; and a therapeutic gas channel in fluid communication with the at least one nasal extension, wherein the therapeutic gas channel is configured to direct a therapeutic gas flow toward the at least one nasal extension.

Clause 20. The nasal cannula of Clause 19, wherein the nebulizing gas flow and the therapeutic gas flow are configured to mix in a combining volume.

Clause 21. The nasal cannula of Clause 19 or 20, wherein the gas channel comprises a flow restrictor to proportion flow between the gas channel and the therapeutic gas channel.

Clause 22. The nasal cannula of Clause 21, wherein the flow restrictor comprises an elastomeric member configured to extend at least partially into a tapered portion of the gas channel to adjustably restrict flow in the gas channel.

Clause 23. The nasal cannula of Clause 21, wherein the flow restrictor is configured to proportion flow between the therapeutic gas channel and the gas channel at a ratio of between 4:1 to 10:1.

Clause 24. The nasal cannula of Clause 19-23, wherein the gas channel comprises a baffle, and wherein the gas flow impinges upon the baffle.

Clause 25. The nasal cannula of Clause 19-24, wherein the nebulizer channel comprises a sealing membrane.

Clause 26. The nasal cannula of Clause 19-25, further comprising a pressure relief valve in fluid communication with the therapeutic gas channel.

Clause 27. The nasal cannula of Clause 19-26, wherein the nebulizer channel comprises a cross-sectional area that is smaller than a cross-sectional area of the gas channel.

Clause 28. The nasal cannula of Clause 19-27, wherein the reservoir is removable from the housing volume.

Clause 29. The nasal cannula of Clause 28, wherein the reservoir can be releasably attached to the cannula housing.

Clause 30. The nasal cannula of Clause 29, wherein the nebulizer channel comprises a sealing membrane.

Clause 31. The nasal cannula of Clause 19-30 wherein the reservoir is in fluid communication with a medicament delivery line, wherein the medicament delivery line is configured to deliver medicament into the reservoir volume.

Clause 32. The nasal cannula of Clause 19-31, wherein the reservoir comprises an access port configured to allow manual administration of the medicament into the reservoir volume.

Clause 33. The nasal cannula of Clause 32, wherein the access port is configured to allow introduction of an ampule into the reservoir volume.

Clause 34. The nasal cannula of Clause 19-33, wherein the medicament comprises a liquid medicament.

Clause 35. The nasal cannula of Clause 19-34, wherein the medicament comprises a powdered medicament.

Clause 36. The nasal cannula of Clause 19-35, wherein the cannula housing is configured to be disposed adjacent to the patient's nares.

Clause 37. The nasal cannula of Clause 36, further comprising: a strap coupled to the cannula housing, wherein the strap is configured to be disposed around a patient's head.

Clause 38. The nasal cannula of Clause 19-37, wherein the nebulizing gas flow is in fluid communication with a first nasal extension and the therapeutic gas flow is in fluid communication with a second nasal extension.

Clause 39. A method comprising: introducing at least one nasal extension of a cannula housing into a patient's nares; directing a gas flow through a nebulizer channel toward the at least one nasal extension; drawing a portion of a medicament from a reservoir through the nebulizer channel with the gas flow, wherein the reservoir is disposed within the cannula housing; and directing the gas flow and the portion of the medicament toward the patient's nares via the at least one nasal extension.

Clause 40. The method of Clause 39, further comprising directing the gas flow through the nebulizer channel to create negative pressure to draw the portion of the medicament from the reservoir.

Clause 41. The method of Clause 39 or 40, further comprising pulverizing the portion of the medicament into an aerosol in the nebulizer channel.

Clause 42. The method of Clause 39-41, further comprising introducing turbulence to the gas flow within the nebulizer channel.

Clause 43. The method of Clause 39-42, further comprising introducing the reservoir into the cannula housing.

Clause 44. The method of Clause 43, further comprising releasably attaching the reservoir to the cannula housing.

Clause 45. The method of Clause 39-44, further comprising accessing the medicament from the reservoir through a sealing membrane.

Clause 46. The method of Clause 39-45, further comprising directing a therapeutic gas flow through a therapeutic gas channel toward the patient's nares via the at least one nasal extension.

Clause 47. The method of Clause 46, further comprising proportioning flow between the gas flow and the therapeutic gas flow.

Clause 48. The method of Clause 39-47, further comprising securing the cannula housing around a patient's head via a strap coupled to the cannula housing.

Clause 49. The method of Clause 39-48, wherein the gas flow and the portion of the medicament form a nebulizing gas flow, and wherein the nebulizing gas flow is directed to the patient through a first nasal extension and the therapeutic gas flow is directed to the patient through a second nasal extension.

Clause 50. A method comprising: introducing at least one nasal extension of a cannula housing into a patient's nares; directing a gas flow through a nebulizer channel toward the at least one nasal extension; drawing a portion of a medicament from a reservoir through the nebulizer channel with the gas flow, wherein the reservoir is disposed within the cannula housing; forming a nebulizing gas flow by mixing the portion of the medicament with the gas flow; directing the nebulizing gas flow and the portion of the medicament toward the patient's nares via the at least one nasal extension; and directing a therapeutic gas flow through a therapeutic gas channel toward the patient's nares via the at least one nasal extension.

Clause 49. The method of Clause 48, further comprising mixing the nebulizing gas flow and the therapeutic gas flow in a combining volume.

Clause 50. The method of Clause 48 or 49, further comprising proportioning flow between the gas flow and the therapeutic gas flow.

Clause 51. The method of Clause 48-50, further comprising directing the gas flow through the nebulizer channel to create negative pressure to draw the portion of the medicament from the reservoir.

Clause 52. The method of Clause 48-51, further comprising pulverizing the portion of the medicament into an aerosol in the nebulizer channel.

Clause 53. The method of Clause 48-52, further comprising introducing turbulence to the nebulizing gas flow within the nebulizer channel.

Clause 54. The method of Clause 48-53, further comprising introducing the reservoir into the cannula housing.

Clause 55. The method of Clause 54, further comprising releasably attaching the reservoir to the cannula housing.

Clause 56. The method of Clause 48-55, further comprising accessing the medicament from the reservoir through a sealing membrane.

Clause 57. The method of Clause 48-56, further comprising delivering the medicament into the reservoir via a medicament delivery line.

Clause 58. The method of Clause 57, further comprising dripping the medicament into the reservoir via the medicament delivery line.

Clause 59. The method of Clause 58, further comprising continuously metering the medicament into the reservoir via the medicament delivery line.

Clause 60. The method of Clause 48-59, further comprising introducing the medicament into the reservoir via an access port.

Clause 61. The method of Clause 60, further comprising introducing an ampule comprising the medicament into the reservoir via the access port.

Clause 62. The method of Clause 48-61, further comprising securing the cannula housing around a patient's head via a strap coupled to the cannula housing.

### Further Considerations

In some embodiments, any of the clauses herein may depend from any one of the independent clauses or any one of the dependent clauses. In one aspect, any of the clauses (e.g., dependent or independent clauses) may be combined with any other one or more clauses (e.g., dependent or independent clauses). In one aspect, a claim may include some or all of the words (e.g., steps, operations, means or components) recited in a clause, a sentence, a phrase or a paragraph. In one aspect, a claim may include some or all of the words recited in one or more clauses, sentences, phrases or paragraphs. In one aspect, some of the words in each of the clauses, sentences, phrases or paragraphs may be removed. In one aspect, additional words or elements may be added to a clause, a sentence, a phrase or a paragraph. In one aspect, the subject technology may be implemented without utilizing some of the components, elements, functions or operations described herein. In one aspect, the subject technology may be implemented utilizing additional components, elements, functions or operations.

The present disclosure is provided to enable any person skilled in the art to practice the various aspects described herein. The disclosure provides various examples of the subject technology, and the subject technology is not limited to these examples. Various modifications to these aspects will be readily apparent to those skilled in the art, and the generic principles defined herein may be applied to other aspects.

A reference to an element in the singular is not intended to mean "one and only one" unless specifically so stated, but rather "one or more." Unless specifically stated otherwise, the term "some" refers to one or more. Pronouns in the masculine (e.g., his) include the feminine and neuter gender (e.g., her and its) and vice versa. Headings and subheadings, if any, are used for convenience only and do not limit the invention.

The word "exemplary" is used herein to mean "serving as an example or illustration." Any aspect or design described herein as "exemplary" is not necessarily to be construed as preferred or advantageous over other aspects or designs. In one aspect, various alternative configurations and operations described herein may be considered to be at least equivalent.

A phrase such as an "aspect" does not imply that such aspect is essential to the subject technology or that such aspect applies to all configurations of the subject technology. A disclosure relating to an aspect may apply to all configurations, or one or more configurations. An aspect may provide one or more examples. A phrase such as an aspect may refer to one or more aspects and vice versa. A phrase such as an "embodiment" does not imply that such embodiment is essential to the subject technology or that such embodiment applies to all configurations of the subject technology. A disclosure relating to an embodiment may apply to all embodiments, or one or more embodiments. An embodiment may provide one or more examples. A phrase such an embodiment may refer to one or more embodiments and vice versa. A phrase such as a "configuration" does not imply that such configuration is essential to the subject technology or that such configuration applies to all configurations of the subject technology. A disclosure relating to a configuration may apply to all configurations, or one or more configurations. A configuration may provide one or more examples. A phrase such a configuration may refer to one or more configurations and vice versa.

In one aspect, unless otherwise stated, all measurements, values, ratings, positions, magnitudes, sizes, and other specifications that are set forth in this specification, including in the claims that follow, are approximate, not exact. In one aspect, they are intended to have a reasonable range that is consistent with the functions to which they relate and with what is customary in the art to which they pertain.

In one aspect, the term "coupled" or the like may refer to being directly coupled. In another aspect, the term "coupled" or the like may refer to being indirectly coupled.

Terms such as "top," "bottom," "front," "rear" and the like if used in this disclosure should be understood as referring to an arbitrary frame of reference, rather than to the ordinary gravitational frame of reference. Thus, a top surface, a bottom surface, a front surface, and a rear surface may extend upwardly, downwardly, diagonally, or horizontally in a gravitational frame of reference.

Various items may be arranged differently (e.g., arranged in a different order, or partitioned in a different way) all without departing from the scope of the subject technology. All structural and functional equivalents to the elements of the various aspects described throughout this disclosure that are known or later come to be known to those of ordinary skill in the art are expressly incorporated herein by reference and are intended to be encompassed by the claims. Moreover, nothing disclosed herein is intended to be dedicated to the public regardless of whether such disclosure is explicitly recited in the claims. No claim element is to be construed under the provisions of 35 U.S.C. §112, sixth paragraph, unless the element is expressly recited using the phrase "means for" or, in the case of a method claim, the element is recited using the phrase "step for." Furthermore, to the extent that the term "include," "have," or the like is used, such term is intended to be inclusive in a manner similar to the term "comprise" as "comprise" is interpreted when employed as a transitional word in a claim.

The Title, Background, Summary, Brief Description of the Drawings and Abstract of the disclosure are hereby incorporated into the disclosure and are provided as illustrative examples of the disclosure, not as restrictive descriptions. It is submitted with the understanding that they will not be used to limit the scope or meaning of the claims. In addition, in the Detailed Description, it can be seen that the description provides illustrative examples and the various features are grouped together in various embodiments for the purpose of streamlining the disclosure. This method of disclosure is not to be interpreted as reflecting an intention that the claimed subject matter requires more features than are expressly recited in each claim. Rather, as the following claims reflect, inventive subject matter lies in less than all features of a single disclosed configuration or operation. The following claims are hereby incorporated into the Detailed Description, with each claim standing on its own as a separately claimed subject matter.

The claims are not intended to be limited to the aspects described herein, but is to be accorded the full scope consistent with the language claims and to encompass all legal equivalents. Notwithstanding, none of the claims are intended to embrace subject matter that fails to satisfy the requirement of 35 U.S.C. §101, 102, or 103, nor should they be interpreted in such a way.

## Claims

1. A nasal cannula, comprising:
a cannula housing defining a housing volume, the cannula housing comprising at least one nasal extension extending from the cannula housing, wherein the at least one nasal extension is configured to be disposed within a patient's nares;
a reservoir disposed at least partially within the housing volume, wherein the reservoir comprises a reservoir volume configured to dispense a medicament;
a nebulizer channel in fluid communication with the reservoir volume and the at least one nasal extension;
a gas channel in fluid communication with the nebulizer channel, wherein the gas channel is configured to draw a portion of the medicament from the reservoir through the nebulizer channel with the gas flow and form a nebulizing gas flow directed toward the at least one nasal extension; and
a therapeutic gas channel in fluid communication with the at least one nasal extension, wherein the therapeutic gas channel is configured to direct a therapeutic gas flow toward the at least one nasal extension.

2. The nasal cannula of Claim 1, wherein the nebulizing gas flow and the therapeutic gas flow are configured to mix together to form a combining volume.

3. The nasal cannula of Claim 1, wherein the nebulizing gas flow is in fluid communication with a first nasal extension and the therapeutic gas flow is in fluid communication with a second nasal extension.

4. The nasal cannula of Claim 1, wherein the gas channel comprises a baffle, wherein the gas flow impinges upon the baffle.

5. A method comprising:
introducing at least one nasal extension of a cannula housing into a patient's nares;
directing a gas flow through a nebulizer channel toward the at least one nasal extension;
drawing a portion of a medicament from a reservoir through the nebulizer channel with the gas flow, wherein the reservoir is disposed within the cannula housing; and
directing the gas flow and the portion of the medicament toward the patient's nares via the at least one nasal extension.

6. The method of Claim 5, further comprising directing the gas flow through the nebulizer channel to create negative pressure to draw the portion of the medicament from the reservoir.

7. The method of Claim 5, further comprising pulverizing the portion of the medicament into an aerosol in the nebulizer channel.

8. The method of Claim 5, further comprising introducing turbulence to the gas flow within the nebulizer channel.

9. The method of Claim 5, further comprising introducing the reservoir into the cannula housing.

10. The method of Claim 5, further comprising releasably attaching the reservoir to the cannula housing.

11. The method of Claim 10, further comprising accessing the medicament from the reservoir through a sealing membrane.

12. The method of Claim 5, further comprising directing a therapeutic gas flow through a therapeutic gas channel toward the patient's nares via the at least one nasal extension.

13. The method of Claim 12, further comprising proportioning flow between the gas flow and the therapeutic gas flow.

14. The method of Claim 12, wherein the gas flow and the portion of the medicament form a nebulizing gas flow, and wherein the nebulizing gas flow and the therapeutic gas flow combine in a combining volume of the cannula housing.

15. The method of Claim 12, wherein the gas flow and the portion of the medicament form a nebulizing gas flow, and wherein the nebulizing gas flow is directed to the patient through a first nasal extension and the therapeutic gas flow is directed to the patient through a second nasal extension.
